# EUROPÄISCHE PATENTANMELDUNG

(11) **EP 4 477 314 A2**
(43) Veröffentlichungstag der Anmeldung: **18.12.2024**
(21) Anmeldenummer: 24173387.2
(22) Anmeldetag: 30.04.2024
(51) Int. Cl.: B01L 3/00

(54) **GLASELEMENT UND SYSTEM ZUR UNTERSUCHUNG VON BIOLOGISCHEM MATERIAL SOWIE VERFAHREN ZU DESSEN HERSTELLUNG**

(30) Priorität: 15.06.2023 DE 102023115623
(71) Anmelder: SCHOTT AG, 55122 Mainz (DE)
(72) Erfinder: BLASS, Johanna, 55122 Mainz (DE); MANGOLD, Stephanie, 55122 Mainz (DE); SOHR, Oliver, 55122 Mainz (DE)
(74) Vertreter: Schott Corporate IP

(57) **Zusammenfassung**

Glaselement (1) zur Aufnahme und/oder Weiterleitung von biologischem Material (60), insbesondere menschlichen oder tierischen oder pflanzlichen Zellen und/oder Zellbestandteilen, insbesondere Proteinen und/oder DNA und/oder RNA, mit einer Vielzahl von Mikrokanälen (2), welche eine Oberfläche (U) des Glaselements (1) mit der gegenüberliegenden Oberfläche (O) des Glaselements (1) verbinden oder in einem Sackloch enden. Die Mikrokanäle (2) verjüngen sich von der Unterseite (U) in Richtung zur Oberseite (O). Insbesondere sind Mikrokanäle (2) zumindest bereichsweise trichterförmig ausgebildet.

Ebenso umfasst ist ein System (10) mit einem solchen Glaselement (1), das zur ortsaufgelösten Untersuchung von biologischem Material (60) und/oder zum Einzelmolekülnachweis mittels digitaler PCR geeignet ist.

## Beschreibung

Die Erfindung betrifft ein Glaselement mit Mikrokanälen, das insbesondere für die ortsaufgelöste Detektion und Untersuchung von biologischem Material eingesetzt werden kann. Solch biologisches Material sind beispielsweise menschliche, tierische oder pflanzliche Proteine, Antikörper und/oder DNA einer Zelle, die Bestandteil eines Zellverbundes, wie z.B. Gewebeprobe oder Zellkultur ist. Die Erfindung betrifft ebenso ein System zur Untersuchung von biologischem Material und ein Verfahren zur Herstellung des Glaselements.

Die vorgenannten Untersuchungen werden in der Regel Zell- und/oder DNA-Diagnostik genannt. Das erfindungsgemäße Glaselement erlaubt die ortsaufgelöste Untersuchung des biologischen Materials, im Englischen auch als "spatial diagnostics" oder "spatial biology" bezeichnet. Die vorgenannte Erfindung erlaubt die ortsaufgelöste Untersuchung von Expressionsmustern von DNA und Proteinen einer Zelle, wobei die Information in Bezug auf den Zellverband, wie z.B. Gewebeprobe oder Zellkultur, erhalten bleibt. Sie erlaubt außerdem Einzelmoleküluntersuchungen, beispielsweise von Biomarkern in biologischen oder medizinischen Proben, wie insbesondere Blut, Serum oder Urinproben, Zellüberstände etc..

Glaselemente zum Auftrennen von DNA in flüssigen Medien sind aus der US 20150122656 A1 bekannt. Darin werden Kanäle in eine Glasplatte mit Hilfe von Laserstrahlen gebohrt. Das beschriebene Verfahren beruht auf dem Einbringen von Filamenten in das Glas mittels aufeinander folgender Pulse eines Ultrakurzpulslasers. Durch Verschieben des Fokus des Lasers entstehen aus den Filamenten durch das Substrat hindurchgehende Kanäle mit einem Durchmesser von 1 µm. Es wird angegeben, dass damit besonders glatte Innenwände der erzeugten Kanäle erreicht werden, die von der idealen Zylinderform höchstens vernachlässigbar abweichen.

Für ortsaufgelöste Untersuchungen ist es erstrebenswert, möglichst viele Kanäle in dem Glaselement möglichst nahe beieinander anzuordnen. Ebenso ist es erstrebenswert, den Durchmesser der Kanäle so abzustimmen, dass eine ausreichende Sammeleffizienz für das biologische Material erreicht wird. Jeder Kanal repräsentiert aber eine Schwächung des Glaselements und es muss ein ausreichendes Glasvolumen zwischen den Kanälen vorhanden sein, um eine ausreichende Mindeststabilität zu erreichen, welche eine rationelle Anwendbarkeit des Glaselements sicherstellt. Somit stehen Sammeleffizienz und Ortsauflösung in einem Zielkonflikt.

Die Erfindung löst dieses Problem mittels dem Glaselement entsprechend den unabhängigen Ansprüchen. Das erfindungsgemäße Verfahren stellt ein entsprechendes Glaselement zur Verfügung. Bevorzugte Ausführungsformen ergeben sich aus den abhängigen Ansprüchen.

Die Erfindung umfasst ein Glaselement zur Aufnahme und/oder Weiterleitung von biologischem Material (60), insbesondere menschlichen oder tierischen oder pflanzlichen Zellen und/oder Zellbestandteilen, insbesondere Proteinen und/oder DNA und/oder RNA, mit einer Vielzahl von Mikrokanälen, welche bevorzugt eine Oberfläche des Glaselements mit der gegenüberliegenden Oberfläche des Glaselements verbinden oder in einem Sackloch enden, wobei sich Mikrokanälen von der Unterseite in Richtung zur Oberseite verjüngen. Bevorzugt sind Mikrokanäle zumindest bereichsweise trichterförmig ausgebildet.

Die Unterseite des Glaselements ist diejenige Seite, welche im Anwendungsfall dem zu untersuchenden biologischen Material zugewandt ist. Üblicherweise handelt es sich bei dem Glaselement um ein plattenförmiges Element, ein sogenanntes Slide. Die Mikrokanälen können eine Oberfläche des Glaselements mit der gegenüberliegenden Oberfläche des Glaselements verbinden, insbesondere die Unterseite mit der Oberseite. Die Mikrokanäle sind dann durchgängig. Alternativ ist es aber auch möglich, dass die Mikrokanäle als Sackloch ausgebildet sind, wobei sie eine Seite, definitionsgemäß die Oberseite, nicht durchdringen. Der Boden des Sacklochs befindet sich dann im Bereich und/oder der Nähe der Oberfläche. Die Mikrokanäle weiten sich von der Seite einer Oberfläche des Glaselements in Richtung der gegenüberliegenden Oberfläche auf, bevorzugt sind sie zumindest bereichsweise trichterförmig ausgebildet sind.

Ein vorteilhaftes Glaselement sieht es entsprechend vor, dass der Durchmesser des Kanaleingangs eines Mikrokanals an der Unterseite größer als der Durchmesser des Kanalausgangs oder Kanalendes im Bereich der Oberseite.

Dies kann als Folge der Aufweitung der Mikrokanäle betrachtet werden. Die Kanäle haben auf einer Seite des Glaselements eine Öffnung, deren Durchmesser größer ist als der Durchmesser auf der Öffnung oder des Endes des Sacklochs auf der gegenüberliegenden Seite. Üblicherweise handelt es ich um die Oberflächen um die Hauptseiten eines plattenförmigen Glaselements. Deren Unterseite ist definiert als diejenige Seite, die im Anwendungsfall dem zu untersuchenden biologischen Material zugewandt ist, und die Oberseite ist die gegenüberliegende Seite, die der Untersuchungseinrichtung zugewandt ist. Die Aufweitung des Kanals befindet sich üblicherweise auf der Unterseite und ist mithin dem zu untersuchenden biologischen Material zugewandt. Besonders vorteilhaft haben die Mikrokanäle einen kreisrunden oder ovalen Durchmesser. Mikrokanäle im Sinne der vorliegenden Beschreibung bedeutet, dass deren Durchmesser im Bereich von wenigen Mikrometern bis zu wenigen hundert Mikrometern liegt.

Durch die Aufweitung von Mikrokanälen an der Unterseite des Glaselements und damit seiner prinzipiellen Trichterform wird die Sammeleffizienz für das biologische Material erhöht, während zwischen den Mikrokanälen auf der Oberseite des Glaselements mehr Glasmaterial vorhanden ist, welches die mechanische Stabilität des Glaselements sicherstellt. So können die Öffnungen der Kanäle auf der Unterseite des Glaselements sehr nahe beieinander angeordnet sein und damit eine gute Ortsauflösung erzielt werden, aber immer noch eine gute mechanische Stabilität bereit gestellt werden.

Ebenso möglich und von der Erfindung ist ein Doppeltrichter, bei dem die Aufweitungen von Mikrokanälen auf der Oberseite und auf der Unterseite des Glaselements vorliegen. Im mittleren Bereich der Mikrokanäle sind diese sozusagen eingeschnürt und/oder zylinderförmig ausgebildet und es befindet sich genügend Glasmaterial zwischen diesen Bereichen und somit im Volumen des Glaselements.

Eine vorteilhafte Ausführungsform betrifft ein Glaselement, bei welchem die Mikrokanäle zumindest im Bereich eines Kanaleingangs oder Kanalausgangs im Bereich einer Oberfläche des Glaselements einen Durchmesser von 5 µm bis 200 µm aufweisen, vorteilhaft von 7 µm bis 130 µm, besonders vorteilhaft von 10 µm bis 100 µm.

Durch die Wahl dieser Durchmesser ist es biologischem Material möglich, auf einer Seite des Glaselements, wie beschrieben üblicherweise der Unterseite, in Mikrokanäle einzudringen und in Richtung der gegenüberliegenden Oberfläche transportiert zu werden. Die Auswahl der Durchmesser trägt dazu bei oder bestimmt, dass nur das zu untersuchende biologische Material, typischerweise mit Längen von unter 1 µm, nicht aber unerwünschte größere Zellen und/oder Zellbestandteile in die Mikrokanäle gelangen können.

Ein vorteilhaftes Glaselement sieht vor, dass der Kanaleingang einen Durchmesser von 5 µm bis 200 µm aufweist, bevorzugt von 10 µm bis 100 µm.

Wie beschrieben kann dies ein konischer Bereich sein, der sich in Richtung der gegenüberliegenden Oberfläche verjüngt oder es kann sich an den aufgeweiteten Bereich ein zylindrischer Bereich mit weitestgehend konstantem Durchmesser anschließen oder ein sich Richtung der gegenüberliegenden Oberfläche sich aufweitender Bereich. Ebenso ist es möglich, dass ich an den zylindrischen Bereich ein sich aufweitender Bereich anschließt.

Der Öffnungswinkel des trichterförmigen Bereichs von allen beschriebenen Mikrokanälen beträgt vorteilhaft von 0,1° bis 30°, insbesondere vorteilhaft von 2° bis 18°. Vorteilhaft beträgt die Dicke des Glaselements 0.1 mm bis 3 mm.

Die Auswahl der angegebenen Öffnungswinkel ermöglicht eine effiziente Herstellung, insbesondere mittels der weiter unten beschriebenen Verfahren. Ebenso tragen diese Werte dazu bei, dass das zu untersuchende biologische Material gut vom Kanaleingang in Richtung der anderen Oberfläche transportiert werden kann, ohne dass es zu einer Stauung kommt.

Die Auswahl des Öffnungswinkels erlaubt eine Aufkonzentrierung des zu untersuchenden Materials innerhalb eines Kanals bzw. Separierung zwischen zwei oder mehr Kanälen des biologischen Materials an der Oberseite des Glaselements. Die Aufkonzentrierung ist proportional zum Verhältnis der quadratischen Kanaldurchmesser (Rᵤₙₜₑₙ)²/(R_{oben} )², auch Radienverhältnis genannt, erhöht die Sensitivität der Analyse mittels Fluoreszenzspektroskopie bzw. ermöglicht die gezielte Wechselwirkung mit funktionellen Molekülen an der Kanalwand. Besonders vorteilhaft ist eine Aufkonzentrierung bei einem Radienverhältnis von über 5.

Das erfindungsgemäße Verfahren, das im Folgenden beschrieben wird, ermöglicht die Herstellung in diesem breiten Bereich, vorteilhaft aber insbesondere bei den sehr geringen Dicken.

Entsprechend den voranstehenden Ausführungen sieht ein vorteilhaftes Glaselement vor, dass den trichterförmigen Bereich im Bereich des Kanaleingangs ein zylinderförmiger oder trichterförmiger Bereich insbesondere im Bereich des Kanalausgangs und/oder des Endes des Sacklochs anschließt.

Durch die genannten Merkmale ist es möglich, dass das Glaselement eine sehr hohe Dichte von Mikrokanälen aufweisen kann. Die Dichte von Mikrokanälen wird auch Mikrokanaldichte genannt und gibt die Anzahl von Mikrokanälen pro Flächeneinheit auf der Oberfläche des Glaselements an, beispielsweise die Anzahl der Mikrokanäle pro Quadratmillimeter. Da der Kanaleingang immer einem Kanalausgang oder Ende eines Sacklochs zugeordnet ist, ist die Mikrokanaldichte auf der Oberseite des Glaselements gleich der Mikrokanaldichte auf der Unterseite des Glaselements. Daher kann man von einer generellen Mikrokanaldichte sprechen.

Besonders vorteilhaft weist ein Glaselement entsprechend der Erfindung eine Mikrokanaldichte vom wenigstens 20 / mm² oder wenigstens 50 / mm² auf, insbesondere mindestens 400 / mm², insbesondere mindestens 10000 / mm² als Untergrenze auf. Die Obergrenze kann jeweils vorteilhaft höchstens 16000 / mm² betragen.

Besonders vorteilhaft ist ein Glaselement, wobei die Kanalwand eine Struktur mit einer Vielzahl von abgerundeten, kalottenförmigen Vertiefungen beinhaltet, die insbesondere eine Tiefe von weniger als 5 µm aufweisen und insbesondere eine Ausdehnung von insbesondere 5 bis 20 µm aufweisen, vorteilhaft beträgt die Rauigkeit von 50 nm bis 1 µm. Der Wert bezieht sich auf den Mittenrauwert Ra.

Die Erfinder haben erkannt, dass es vorteilhaft ist, wenn die Wand der Mikrokanäle überraschender Weise nicht möglichst glatt ausgebildet ist, sondern eine Struktur aufweist. Die genannte Tiefe und Rauigkeit repräsentieren vorteilhafte Ausführungsformen. Mit Tiefe ist dabei die Vertiefung in Richtung senkrecht zur Achse des Kanals gemeint, mit Ausdehnung die Länge der Vertiefungen parallel zur Achse des Kanals.

Diese Strukturen können durch das weiter unten beschriebene Ultrakurzpulslaser-Herstellungsverfahren mit anschließendem Ätzschritt rationell hergestellt werden.

Diese, kalottenförmigen Vertiefungen stellen den Vorteil bereit, dass wenn Gel in die Mikrokanäle eingefüllt wird, dieses einerseits gut einfüllbar ist, aber ebenfalls auch gut in den Kanälen haften bleibt.

Ein weiterer besonderer Vorteil der Struktur der Kanalwand, insbesondere der kalottenförmigen Vertiefungen, ist die Einstellbarkeit des elektroosmotischen Flusses (EOF). Der elektroosmotische Fluss ist eine durch Ladungen induzierte Bewegung von Flüssigkeiten entlang einer polaren Oberfläche in einem elektrischen Feld, beispielweise eine Pufferlösung in einer Glaskapillare.

Die Auswahl der Rauigkeit erlaubt die Einstellbarkeit des Beitrags der EOF zur Bewegung des biologischen Materials in den Kanälen. Eine größere Rauigkeit der polaren Oberfläche führt zu einer Verringerung der Geschwindigkeit des EOF und eventuellen Umkehr der Bewegungsrichtung des polaren biologischen Materials im Glaselement im Vergleich zu einer Oberfläche mit geringerer Rauigkeit.

Die Auswahl der Kanalwandrauigkeit mit gleichzeitig einstellbaren Öffnungswinkel erlaubt das selektive Verschließen des Kanals mittels gegenläufigem EOF und angelegtem elektrischen Feld.

Ein vorteilhaftes Glaselement sieht es vor, dass die Kanalausgänge eine Ausgangsmatrix bilden, in welcher jedem Kanalausgang die Position des Kanaleingangs zuordenbar ist.

Insbesondere dadurch ist es möglich, eine ortsaufgelöste Untersuchung von biologischem Material durchzuführen. Dies insbesondere, da die Kanalausgänge oder alternativ die Positionen der Sacklöcher eine Ausgangsmatrix bilden, in welcher jedem Kanalausgang die Position des Kanaleingangs zuordenbar ist.

Dies bedeutet mit anderen Worten, dass die Position der Eingangsmatrix der Position der Ausgangsmatrix zuordenbar ist. Im einfachsten Fall stimmt der Mittelpunkt eines Kanaleingangs auf der Unterseite des Glaselements wenigstens im Wesentlichen mit der Position des Kanalausgangs auf der Oberseite des Glaselements oder dem Ende des Sacklochs überein. Dies entspricht einer Ausführung der Mikrokanäle, in denen die Achse von Kanälen senkrecht durch die Hauptflächen des Glaselements verlaufen. Es ist aber auch möglich, dass die Kanalachsen schräg durch die Hauptflächen des Glaselements verlaufen. Aber auch in diesem Fall ist die Position des Kanaleingangs der Position des Kanalausgangs oder des Endes des Sacklochs zuordenbar, so dass eine Analyse des zu untersuchenden biologischen Materials am Kanalausgang oder am Ende des Sacklochs eindeutig auf dessen Position bezüglich der Eingangsmatrix und/oder des Kanaleingangs Herkunft schließen lässt.

Vorteilhaft umfasst das Glaselement zumindest im Bereich der Mikrokanäle ein Glas, dass sich dadurch auszeichnet, dass es eine geringe Eigenfluoreszenz im Wellenlängenbereich von 300 nm bis 700 nm aufweist.

Die Eigenfluoreszenz sollte geringer sein als 10⁻⁶ der eingestrahlten Lichtintensität. Durch die geringe Eigenfluoreszenz stört das Fluoreszenzlicht nicht die optische Untersuchung des biologischen Materials. Geeignete Bereiche der Eigenfluoreszenz sind beispielsweise von 10⁻⁸ bis 10⁻⁶ der eingestrahlten Lichtintensität.

Besonders geeignete Glaszusammensetzungen werden in den Beispielen weiter unten genannt. Besonders geeignet sind in Allgemeinen Gläser, welche sich durch eine hohe chemische Beständigkeit und/oder hohe Wasserbeständigkeit auszeichnen. Insbesondere vorteilhaft sind alkalifreie Gläser.

Geeignete Glasmaterialien können insbesondere alkalifreie Gläser und Borosilikatglas sein. Insbesondere , im Handel befindlich Gläser mit den Produktbezeichnungen AF32, AF35, AS87, D263, D263T, B270, MEMPAX, Willow, G-Leaf, EN-A1, BDA-E in Betracht.

Gemäß einer beispielhaften Ausführungsform umfasst die Zusammensetzung des Glases folgende Bestandteile in Gewichtsprozent:

| Zusammensetzung | (Gew.-%) |
|---|---|
| SiO₂ | 63-85 |
| Al₂O₃ | 0-10 |
| B₂O₃ | 5-20 |
| Li₂O + Na₂O + K₂O | 2-14 |
| MgO + CaO + SrO + BaO + ZnO | 0-12 |
| TiO₂ + ZrO₂ | 0-5 |
| P₂O₅ | 0-2 |

Gemäß einer weiteren beispielhaften Ausführungsform umfasst die Zusammensetzung des Glases folgende Bestandteile:

| Zusammensetzung | (Gew.-%) |
|---|---|
| SiO2 | 60-84 |
| Al₂O₃ | 0-10 |

| Zusammensetzung | (Gew.-%) |
|---|---|
| B₂O₃ | 3-18 |
| Li₂O + Na₂O + K₂O | 5-20 |
| MgO + CaO + SrO + BaO + ZnO | 0-15 |
| TiO₂ + ZrO₂ | 0-4 |
| P₂O₅ | 0-2 |

In einer anderen Ausführungsform umfasst die Zusammensetzung des Glases folgende Bestandteile:

| Zusammensetzung | (Gew.-%) |
|---|---|
| SiO₂ | 58-65 |
| Al₂O₃ | 14-25 |
| B₂O₃ | 6-10,5 |
| MgO + CaO + SrO + BaO + ZnO | 8-18 |
| ZnO | 0-2 |

Eine weitere geeignete Zusammensetzung des Glases ist gegeben durch:

| Zusammensetzung | (Gew.-%) |
|---|---|
| SiO₂ | 50-81 |
| Al₂O₃ | 0-5 |
| B₂O₃ | 0-5 |
| Li₂O + Na₂O + K₂O | 5-28 |
| MgO + CaO + SrO + BaO + ZnO | 5-25 |
| TiO₂ + ZrO₂ | 0-6 |
| P₂O₅ | 0-2 |

Gemäß einer weiteren Ausführungsform umfasst die Zusammensetzung des Glases folgende Bestandteile:

| | |
|---|---|
| SiO₂ | 52 - 66 |
| B₂O₃ | 0 - 8 |
| Al₂O₃ | 15 - 25 |
| MgO + CaO + SrO + BaO + ZnO | 0 - 6 |
| ZrO₂ | 0 - 2.5 |
| Li₂O + Na2O + K2O | 4 - 30 |
| TiO₂ + CeO₂ | 0 - 2.5 |

Für alle vorgenannten Glaszusammensetzungen gilt, dass gegebenenfalls färbende Oxide zugegeben werden können, wie Nd₂O₃, Fe₂O₃, CoO, NiO, V₂O₅, MnO2, CuO, Cr₂O₃.

0 - 2 Gew.-% As₂O₃, Sb₂O₃, SnO₂, SOs, Cl, F und/oder CeO₂ können als Läutermittel zugegeben werden, und die Gesamtmenge der Gesamtzusammensetzung beträgt jeweils 100 Gew.-%.

Ebenso kann es vorteilhaft sein und ist von der Erfindung umfasst, wenn die Oberflächenenergie der Wandungen von Mikrokanälen so eingestellt ist, dass das zu untersuchende biologische Material von den Wandungen weiter geleitet wird oder das zu untersuchende biologische Material an den Wandungen anhaftet. Die Oberflächenenergie kann beispielsweise durch Beschichtungen eingestellt werden.

Ein vorteilhaftes Glaselement sieht daher vor, dass zumindest Wandungen der Mikrokanäle beschichtet sind, insbesondere mit Beschichtungen, welche das zu untersuchende biologische Material abstoßen oder an welchen das zu untersuchende biologische Material anhaftet. Vorzugsweise umfasst die Beschichtung insbesondere zur Optimierung der Oberflächenenergie zumindest bereichsweise Polymere, Silane, Oligonucleotide, Antikörper, Proteine, Antigene oder Nitrocellulose oder anorganischen Beschichtungen, insbesondere metallische Partikel, insbesondere Gold und/oder, Eisen oder Polymer basierte Nanopartikel oder Mikropartikel oder Kombinationen der Genannten.

Wie voran beschrieben ist es möglich, dass die Beschichtung nur in Abschnitten der Mikrokanäle vorliegt, beispielsweise am Kanaleingang, oder sich unterschiedliche Beschichtungen im Kanal befinden, insbesondere unterschiedlich entlang der Kanalachse.

Die Beschichtung kann üblicherweise Dicken von einer Molekül-Monolage oder Beschichtungen mit Dicken im nm-Bereich bis zur vollständigen Füllung des Kanals aufweisen. Sie wird im einfachsten Fall über Tauchbeschichtungsverfahren aufgebracht. Besonders bei geringen Kanaldurchmessern eignet sich eine Beschichtung mit Hilfe von Vakuumprozessen, z.B. Chemical Vapor Deposition (CVD) oder Atomic Layer Deposition ALD). Es kann vorteilhaft sein, die Beschichtung selektiv in den Kanälen anzubringen, z.B. durch ein Lift-off Material außerhalb der Kanäle oder selektives Sputtern auf die Oberfläche des Glaselements.

Die genannte vorteilhafte Struktur des Wandung der Mikrokanäle wirkt besonders vorteilhaft mit den Beschichtungen und/oder Füllungen zusammen, insbesondere da die Struktur die Oberfläche die Fläche der Kanalwandung erhöht. Bei anhaftenden Beschichtungen wird dann mehr Fläche für zu untersuchenden biologisches Material geboten, bei weiterleitenden Beschichtungen unterdrückt oder verhindert die Beschichtung das Zusetzen der Strukturen mit biologischem Material und/oder trägt zumindest dazu bei. Bei Füllungen kann die Haftung der Füllung in den Kanälen gesichert oder zumindest verbessert werden.

In einem vorteilhaften Glaselement sind die Mikrokanäle mit einem Gel gefüllt. Dieses weist vorteilhaft Poren auf. Je nach Wahl und/oder Ausgestaltung des Gels können die Poren unterschiedliche Durchmesser haben, so dass sie biologisches Material unterschiedlicher Größe aufnehmen und/oder weiterleiten können.

Im Sinne der Erfindung geeignet sind insbesondere Polyacrylamid-Gel und/oder Agarose-Gel. Agarose-Gel weist üblicherweise größere Poren auf als Polyacrylamid-Gel und kann insbesondere für die Analyse von DNA und/oder größeren Proteinen eingesetzt werden. Polyacrylamid-Gel mit seinen i.d.R. kleineren Poren wird üblicherweise für die Analyse von kleineren Proteinen eingesetzt.

Das Gel, insbesondere die genannten Gele, können im Sinne der Erfindung als Trägergel in einem Elektrophorese-Prozess eingesetzt werden, üblicherweise auch Gelelektrophorese genannt. Mit dieser können insbesondere verschiedene kleine Moleküle, insbesondere DNA, RNA und Proteine, voneinander getrennt und mit nachfolgenden Schritten analysiert werden. Im vorliegenden Fall umfass der Begriff der Gelelektrophorese im Allgemeinen die Weiterleitung und/oder Bewegung der zu untersuchenden Substanzen durch das Gel, eine Auftrennung ist nicht zwangsweise erforderlich.

Die zu trennenden und/oder weiterzuleitenden Moleküle bewegen sich dabei durch das Trägergel, das sich vorteilhaft in den Mikrokanälen befindet. Die Bewegung kann wie weiter beschrieben durch das Anlegen eines elektrischen Felds unterstützt werden. Je nach Größe und Ladung und/oder Stärke des angelegte elektromagnetischen Felds wandern die Moleküle unterschiedlich weit und/oder schnell.

Bei der klassischen Elektrophorese bilden sie ein charakteristisches Bandenmuster innerhalb des Mikrokanals. Die Moleküle können durch eine Färbung sichtbar gemacht machen, die charakteristische Färbung eines Moleküls kann dem Molekül zugeordnet und das Molekül so identifiziert werden.

Bei den erfindungsgemäßen Glaselementen, insbesondere bei dünnen Glaselementen, ist es aber darüber hinaus vorgesehen, dass das Gel und/oder alternative Substanzen zur elektrophoretischen Weiterleitung des zu untersuchenden Materials von der Unterseite des Glaselements zur Oberseite des Glaselements dient. Die Analytik erfolgt dann auf der Oberseite, z.B. mittels Fluoreszenzspektroskopie oder durch DNA-Sequenzierung mittels Nanoporen. Dies sind Standardverfahren und werden hier nicht weiter im Detail aufgeführt.

Besonders vorteilhaft ist ein Glaselement, wobei sich im Bereich der Kanalausgänge oder am Boden der Sacklöcher zumindest ein DNA- oder RNA-sensitiver Farbstoff befindet, insbesondere eine Mehrzahl von Farbstoffen.

Wird DNA- und/oder RNA aus dem zu untersuchenden biologischen Material durch die Mikrokanäle transportiert, gelangt es zum Kanalausgang und/oder zum Boden des Sacklochs, wenn die Mikrokanäle als solches ausgebildet sind. Dort kann DNA- oder RNA mit einem DNA- oder RNA-sensitiven Farbstoff reagieren, der dort angebracht ist. Die charakteristische Fluoreszenz, insbesondere deren Wellenlänge, weit dann das Vorhandensein des entsprechenden DNA- oder RNA-Strangs nach.

Mögliche vorteilhafte Farbstoffe können beispielsweise sein Ethidiumbromid, Propidium Iodide, Kristallviolet, 4',6-diamidino-2-phenylindole und/oder 7-aminoactinomycin D.

Eine vorteilhafte Ausgestaltung des Glaselements sieht vor, dass sich im Bereich der Kanalausgänge oder am Boden der Sacklöcher Moleküle zur Polymerasekettenreaktion befinden werden, insbesondere DNA, Primer, Nukleotide und/oder das Enzym DNA-Polymerase. Diese Ausführungsform ist besonders geeignet für die Einzelmoleküluntersuchung, bei der wie hierin beschrieben das zu untersuchende Material in den Mikrokanälen und/oder Sacklöchern zunächst vervielfältigt wird. Es ist ersichtlich, dass sich die vorgenannten Moleküle insbesondere auch beim Einbringen des zu untersuchenden Materials in die Mikrokanäle und/oder Sacklöcher in eben diese einbringen lässt.

Diese Ausführungsform hat insbesondere den Vorteil, dass die Vervielfältigung der Zellen und/oder DNA und/oder RNA im zu untersuchenden Material in den Mikrokanälen (einschließlich Sacklöcher) in einem sehr kleinen Volumen stattfindet. Dadurch kann die für die benötigte Vervielfältigung erforderliche Zeit reduziert werden. Ebenso kann eine Untersuchung, einschließlich ein Nachweis, des zu untersuchenden Materials in diesem kleinen Volumen erfolgen, was eine gute Auswertbarkeit, insbesondere gute Signalqualität zur Folge hat. Insbesondere können durch die erhöhte Genauigkeit seltene Populationen identifiziert werden oder subtile Unterschiede zwischen Zellen erkannt werden. Neue Biomarker oder Signaturmoleküle können so identifiziert werden. Außerdem kann mit einer Untersuchung der einzelnen Moleküle bzw. Zellen in der Probe eine Aussage über die Heterogenität der Moleküle bzw. Zellen in der Probe getroffen werden. Insbesondere können individuelle Unterschiede zwischen Patienten sichtbar gemacht werden, um personalisierte Behandlungsansätze zu entwickeln, die auf den spezifischen Eigenschaften der Zellen bzw. Moleküle basieren. Insgesamt wird damit eine rationelle und zuverlässige Untersuchung zur Verfügung gestellt, die Anwendungspotentiale in der personalisierten Medizin aufweist.

Das beschriebene Glaselement ist sozusagen das Vorprodukt des erfindungsgemäßen Systems, mit welchem die ortsaufgelöste Diagnostik und/oder Einzelmoleküldiagnostik oder Single-Cell Diagnostik durchführbar ist. Das biologische Material kann wie beschrieben in einem angeschlossenen Prozess auf dem Glaselement oder z.B. mittels DNA-Sequenzierung analysiert und/oder vervielfältigt werden. So kann gleichzeitig das Expressionsmuster von Genen und/oder Proteinen und das Verteilungsmuster vDNA-Markern von einzelnen Zellen bestimmt werden, wobei die Ortsinformation im Zellverbands bzw. der Gewebeprobe erhalten bleibt.

Das System zur Untersuchung von biologischem Material, insbesondere menschlichen oder tierischen oder pflanzlichen Zellen und/oder Zellbestandteilen, insbesondere Proteinen undoder DNA und/oder RNA, umfasst ein Glaselement mit einer Vielzahl von Mikrokanälen, insbesondere einem zuvor beschriebenen Glaselement, wobei das Glaselement im Betriebszustand zur Aufnahme und/oder Weiterleitung des biologischen Materials in den oder durch die Mikrokanäle von der Unterseite des Glaselements in den Bereich der Oberseite des Glaselements dient, mit einer Auswerteeinrichtung, mit welcher im Betriebszustand das biologische Material untersuchbar ist. Die Auswerteeinrichtung ist im Sinne der Erfindung dem Glaselement zugeordnet oder nachgeordnet.

Die Auswerteeinheit erfasst im Allgemeinen das Vorhandensein von durch die Mikrokanäle transportiertem und/oder in diesen vorhandenem biologischem Material und/oder dessen Bestandteilen. Insbesondere kann die Auswerteeinheit einen elektronischen Bildsensor umfassen, der insbesondere in der Lage ist, die Eigenfluoreszenz der vorgenannten Farbstoffe aufzunehmen. Die Ausgestaltung der Mikrokanäle als Sacklöcher ist damit ebenso umfasst.

Ein vorteilhaftes System sieht daher vor, dass sich im Bereich des Kanalausgangs (22) und/oder im Sackloch (25) , insbesondere an dessen Boden, zumindest ein DNA oder RNA sensitiver Farbstoff befindet, dessen Farbinformation durch die Auswerteeinrichtung erfassbar ist, insbesondere ortsaufgelöst erfassbar.

Im Falle von DNA-Untersuchungen ist ein üblicher DNA-bindender Farbstoff beispielsweise Ethidiumbromid.

Vorteilhaft umfasst das System einen Probenträger, auf dem im Betriebszustand das zu untersuchende biologische Material angebracht ist. Dies kann üblicherweise ein Probenplättchen oder Slide sein.

Besonders vorteilhaft ist ein System, wobei dem Glaselement eine Transporteinrichtung zugeordnet ist, mit welcher das biologische Material im Betriebszustand in die Mikrokanäle des Glaselements eingebracht wird.

Die Transporteinrichtung ist im Allgemeinen eine Einrichtung, die eine Kraft auf das zu untersuchende biologische Material bewirkt, welche dessen Bewegung und/oder Transport in die Mikrokanäle des Glaselements hinein und in Richtung dessen gegenüberliegender Oberfläche zumindest unterstützt.

Vorteilhaft ist ein System, wobei die Transporteinrichtung eine Unterdruckeinheit umfasst, mit welcher im Betriebszustand Unterdruck an die Ausgangsseite des Glaselements angelegt wird und/oder eine Einrichtung, um ein Potentialgefälle über die Dicke des Glaselement zu erzeugen, so dass zu untersuchendes biologisches Material durch einen lonenfluss oder durch ein elektrisches Feld in die Mikrokanäle eingebracht und/oder durch diese transportiert wird.

Da DNA aufgrund ihrer Phosphatreste grundsätzlich negativ geladen ist, wandert sie in der Gelelektrophorese zur Anode. Auf der Oberseite eines vorteilhaften Glaselements ist daher besonders vorteilhaft eine Anode angebracht oder die Oberseite ist einer Anode zugeordnet. Befindet sich auf der Unterseite oder in der Nähe der Unterseite eine Kathode, entsteht durch das Anlegen von Spannung ein elektrisches Feld und die Anionen wandern zur Anode und die Kationen wandern zur Kathode.

Bei der DNA-Untersuchung ist es natürlich möglich, die DNA-Abschnitte vorab und/oder in den Kanälen des Glaselements zu vervielfältigen, insbesondere mittels Polymerase-Kettenreaktion.

Je nach zu untersuchendem biologischen Material ist es natürlich auch möglich und von der Erfindung umfasst, die Anode auf der Unterseite des Glaselements vorzusehen und die Kathode auf der Oberseite und/oder jeweils diesen jeweils zuzuordnen. Andere Anordnungen sind ebenso möglich und von der Erfindung umfasst. Beispielsweise kann es auch ausreichend sein, dass das Medium, in welchem sich das zu untersuchende biologische Material befindet, durch die Transporteinrichtung in Bewegung gesetzt wird.

Ebenso vorteilhaft umfasst die Transporteinrichtung eine Rakeleinheit, mit welcher zu untersuchendes biologisches Material durch Verstreichen in die Mikrokanäle, insbesondere die Sacklöcher eingebracht werden kann.

Ein vorteilhaftes System umfasst als Transporteinrichtung ein mikrofluidisches System, das das zu untersuchende biologische Material mittels fluider Strömung in die Sacklöcher und/oder Mikrokanäle einbringt.

Besonders vorteilhaft wir das System so weitergebildet, dass die Auswerteeinrichtung eine geschlossene oder nanoporöse Membran insbesondere aus einem Nichtleiter oder einem Halbleiter oder der Kombination aus nichtleitenden, leitenden und/oder halbleitenden Bestandteilen insbesondere Glas, Silizium, Graphen und/oder Biomolekülen insbesondere Lipidmolekülen, DNA-Origami Strukturen oder Transmembranproteine auf der Ausgangsseite der Mikrokanäle umfasst mit welcher das biologische Material auf insbesondere die Sequenz oder das Bindungsverhalten oder die Größe oder die optischen Eigenschaften insbesondere die Fluoreszenz untersucht werden kann.

In einer alternative Ausgestaltung umfasst das System eine Auswerteeinrichtung in Form einer geschlossenen oder nanoporöse Membran, insbesondere aus einem Nichtleiter oder einem Halbleiter oder der Kombination aus nichtleitenden, leitenden und/oder halbleitenden Bestandteilen insbesondere Glas, Silizium, Graphen und/oder Biomolekülen insbesondere Lipidmolekülen oder Transmembranproteine, auf der Oberseite der Mikrokanäle. Mit dieser wird das zu untersuchende biologische Material auf insbesondere die Sequenz oder das Bindungsverhalten oder die Größe oder die optischen Eigenschaften, insbesondere die Fluoreszenz, untersucht.

Wie beschrieben können die Mikrokanäle mit einem Gel gefüllt sein, um insbesondere den Transport zu unterstützen. Das Gel selbst kann einen Farbstoff enthalten, der an das zu untersuchende biologische Material bindet. Im Falle von DNA-Untersuchungen einen DNA-bindenden Farbstoff wie beispielsweise Ethidiumbromid. Ebenso ist es möglich, dass die Probe sich in einem Probenpuffer befindet, der seinerseits einen Farbstoff enthält, beispielsweise Kristallviolett. Alternativ kann das Gel nach der Elektrophorese mit einem DNA-bindenden Farbstoff gefärbt werden, beispielsweise mit Methylenblau, Stains-all oder Ethidiumbromid. Bei Fluoreszenzfarbstoffen wie Ethidiumbromid ist oftmals eine Beleuchtung des Agarosegels mit UV-Licht und ein UV-Filter an der Fotokamera erforderlich. RNA-Untersuchungen sind selbstverständlich ebenso möglich.

Ein vorteilhaftes System sieht vor, dass im Betriebszustand biologisches Material in Mikrokanälen und/oder Sacklöchern vervielfältigt wird, oft digital PCR genannt. Insbesondere sind im Bereich der Kanalausgänge oder am Boden der Sacklöcher Moleküle zur Poly-merasekettenreaktion vorhanden.

Wenn die Sacklöcher Moleküle zur Polymerasekettenreaktion (PCR) enthalten, können in dem zu untersuchenden Material enthaltene DNA- oder RNA-Moleküle viervielfältigt werden. Dazu gehören beispielsweise DNA, Primer, Nukleotide und das Enzym DNA-Polymerase.

Aufgrund der temperaturstabilen Eigenschaften des Glaselements sind neben der Raumtemperatur-PCR, die üblicherweise bei bis zu 40°C durchgeführt wird, die Standard-PCR mit bis zu knapp 100°C möglich. Bei diesen Temperaturen ist kein negativer Einfluss auf die räumliche Information durch die thermische Ausdehnung oder Verschiebung der Position der Sacklöcher gegeneinander erwartbar, im Gegensatz zu Kunststoffen. Des Weiteren hat die gute Wärmeleitfähigkeit von Glas im Vergleich zu Kunstoffen hat den Vorteil, dass sich die Wärme gleichmäßig im Glaselement verteilt und eine homogene PCR über die gesamte Probe gewährleistet werden kann. Des Weiteren hat Glas im Vergleich zu Kunststoff den Vorteil einer niedrigen Eigenfluoreszenz, so dass eine hohe Signalstärke im Vergleich zum Hintergrund erreicht wird.

Da wie beschrieben die Ausgangsmatrix der Mikrokanäle auf der Oberseite des Glaselements der Eingangsmatrix der Mikrokanäle auf der Unterseite des Glaselements zuordenbar ist, ist das beschriebene System in der Lage, aus der Position eines Untersuchungsergebnisses auf der Oberseite auf die Position des Eingangs des entsprechenden biologischen Materials auf der Unterseite zu schließen. Dem Ort eines Mikrokanals auf dem Glaselement ist sozusagen ein Ort der Herkunft des biologischen Materials in der Probe zuordenbar. Damit ist das erfindungsgemäße Glaselement und/oder das erfindungsgemäße System dazu in der Lage, eine ortsaufgelöste Untersuchung von Proben von biologischem Material zu ermöglichen. Dies nennt man auch "Spatial Biology" oder "Spatial Diagnostics".

Das beschriebene Glaselement lässt sich rationell und in großer Stückzahl durch das im Folgenden erläuterte erfindungsgemäße Verfahren herstellen. Das Verfahren umfasst die Verfahrensschritte
- Bereitstellen eines Ultrakurzpuls-Lasers,
- Bereitstellen eines Glaselement-Grundkörpers,
- ein gepulster Laserstrahl des Ultrakurzpulslasers wird auf den Glaselement-Grundkörper gerichtet, wobei
- die Wellenlänge des Laserstrahls und das Material des Glaselement-Grundkörpers so aufeinander abgestimmt sind, dass das Werkstück für den Laserstrahl im Wesentlichen transparent ist, und wobei
- der Laserstrahl zu einem in Strahlrichtung langgestreckten Fokusbereich fokussiert wird, welcher mindestens teilweise innerhalb des Glaselement-Grundkörpers liegt, wobei
- die Intensität des Laserstrahls und die Ausdehnung des Fokus so groß sind, dass der Laserstrahl im Werkstück eine filamentförmige Schädigung hinterlässt, und wobei eine Vielzahl solcher filamentförmiger Schädigungen eingefügt werden,
- Aufätzen der filamentförmigen Schädigungen zu Mikrokanälen.

Der Glaselement-Grundkörper kann im Allgemeinen ein Glasplättchen der gewünschten Dimensionen sein. Ein gepulster Laserstrahl des Ultrakurzpulslasers wird auf den Glaselement-Grundkörper gerichtet, wobei die Wellenlänge des Laserstrahls und das Material des Glaselement-Grundkörpers so aufeinander abgestimmt sind, dass das Werkstück für den Laserstrahl im Wesentlichen transparent ist.

Der Laserstrahl wird zu einem in Strahlrichtung langgestreckten Fokusbereich fokussiert, welcher mindestens teilweise innerhalb des Glaselement-Grundkörpers liegt. Dazu können verschiedene optische Aufbauten verwendet werden, beispielsweise Bessel-Optiken mit Verwendung eines Axikons, Optiken mit chromataischer Aberration oder Optiken mit sphärischer Aberration.

Die Intensität des Laserstrahls und die Ausdehnung des Fokus werden so groß eingestellt, dass der Laserstrahl im Werkstück eine filamentförmige Schädigung hinterlässt, wobei eine Vielzahl solcher filamentförmiger Schädigungen eingefügt werden. Die filamentförmig Schädigung repräsentiert sozusagen eine fadenförmige Schädigung im Glas, die in einem darauffolgenden Verfahrensschritt zu Mikrokanälen aufgeätz werden.

Die filamentförmige Schädigung entsteht durch einen nichtlinearen optischen Effekt mit Selbstfokusierung des Laserstrahls. Dazu sind ultrakurze Laserpulse notwending, die im ps-Bereich liegen.

Ein vorteilhaftes Verfahren sieht vor, dass zur Erzeugung einer filamentförmigen Schädigung eine Folge von Laserpulsen eingesetzt wird.

In diesem sogenannten Burst-Betriebsmodus wird die Laserenergie nicht als Einzelpuls abgegeben, sondern als Folge kurz hintereinander abgegebener Pulse, die gemeinsam ein Pulspaket, einen sogenannten Burst, bilden. Ein solches Pulspaket weist typischerweise eine etwas größere Energie auf, als ein Einzelpuls im üblichen Single-Shot-Betrieb. Die Pulse eines Bursts selbst beinhalten aber dafür deutlich weniger Energie als ein Einzelpuls. Hinsichtlich der Pulse innerhalb eines Bursts kann vorgesehen sein, dass die Pulsenergien flexibel einstellbar sind, insbesondere, dass die Pulsenergien entweder im Wesentlichen konstant bleiben oder dass die Pulsenergien zunehmen oder dass die Pulsenergien abnehmen.

Eine geeignete Laserquelle kann beispielsweise ist ein Neodym-dotierter Yttrium-Aluminium-Granat-Laser mit einer Wellenlänge von 1064 Nanometern sein. Die Laserquelle erzeugt beispielsweise einen Rohstrahl mit einem (1/e2)-Durchmesser von 12 mm, als Optik kann eine Bikonvex-Linse mit einer Brennweite von 16 mm zum Einsatz kommen. Zur Erzeugung des Rohstrahls kann ggf. eine geeignete strahlformende Optik, wie beispielsweise ein Galilei-Teleskop zum Einsatz kommen.

Die Laserquelle kann insbesondere mit einer Repetitionsrate arbeiten, welche zwischen 1 kHz und 1000 kHz, vorzugsweise zwischen 2 kHz und 100 kHz, besonders bevorzugt zwischen 3 kHz und 200 kHz.

Die Repetitionsrate und/oder die Scangeschwindigkeit kann dabei so gewählt werden, dass der gewünschte Abstand benachbarter filamentförmiger Schädigungen erreicht wird.

Die geeignete Pulsdauer eines Laserpulses kann insbesondere in einem Bereich von weniger als 100 Pikosekunden, vorteilhaft bei weniger als 20 Pikosekunden liegen.

Die typische Leistung der Laserquelle liegt dabei besonders günstig in einem Bereich von 20 bis 300 Watt. Die Laserenergie liegt im Wesentlichen unterhalb der Ablationsenergie des Substrats. Um die filamentförmigen Schädigungen zu erzielen, wird gemäß einer vorteilhaften Weiterbildung der Erfindung eine Pulsenergie im Burst von mehr als 400 Mikrojoule eingesetzt, ferner vorteilhaft eine gesamte Burstenergie von mehr als 500 Mikrojoule.

Beim Betrieb des Ultrakurzpuls-Lasers im Burst-Modus ist die Repetitionsrate die Wiederholrate der Abgabe von Bursts. Die Pulsdauer ist im Wesentlichen unabhängig davon, ob ein Laser im Einzelpulsbetrieb oder im Burst-Mode betrieben wird. Die Pulse innerhalb eines Bursts weisen typischerweise eine ähnliche Pulslänge auf, wie ein Puls im Einzelpulsbetrieb. Die Burstfrequenz kann im Bereich von 15 MHz bis 90 MHz liegen, bevorzugt im Bereich von 20 MHz bis 85 MHz liegen und beträgt beispielsweise 50 MHz und die Anzahl der Pulse im Burst kann zwischen 1 und 10 Pulsen, z.B. 6 Pulsen liegen.

Vorteilhaft ist ein Verfahren, wobei der langestreckte Fokusbereich einen Bereich mit höchster Energie und einen daran anschließenden aufgeweiteten Anteil umfasst, wobei der langestreckte Fokusbereich so platziert wird, dass der aufgeweitete Anteil in einem Oberflächenbereich des Glaselement-Grundkörpers liegt.

Auf diese Weise lassen sich besonders vorteilhaft die sich verjüngenden Mikrokanäle erzeugt werden. Die filamentförmige Vorschädigung wird sozusagen mit einem Schädigungsbereich erzeugt, der im nachfolgenden Ätzschritt die Aufweitung am Kanaleingang zur Folge hat.

Ein besonders vorteilhaftes Verfahren beinhaltet, dass die filamentförmigen Schädigungen zumindest eine Oberfläche des Glaselement-Grundkörpers nicht erreichen und die Öffnung zu dieser Oberfläche durch den nachfolgenden Ätzschritt erfolgt.

Dies kann ebenfalls durch die Wahl der Fokuslage und damit der Intensität des Laserstrahls erreicht werden. Es wird vermutet, da es sich beim Erzeugen der filamentförmigen Schädigung um einen nichtlinearen Effekt handelt, dass eine lokale Strahlintensität erreicht werden muss, um überhaupt die Schädigung im Glas zu erzeugen. Dies bedeutet, das bei Unterschreiten dieser kritischen Schwelle de facto keine Schädigung oder zumindest keine ausreichende Schädigung im Glas vorliegt, um ein filament zu bilden. Erst der nachfolgende Ätzprozess weitet die filamentförmigen Schädigungen auf und erzeugt sozusagen den Durchbruch durch die betreffende Oberfläche des Glaselement-Grundkörpers. Die Erfinder haben erkannt, dass sich die Matrix der Kanalausgänge der Mikrokanäle dadurch besonders genau herstellen lässt. Besonders genau heißt in diesem Sinne, dass die Varianz der Durchmesser der Kanalausgänge gering ist und/oder höchstens eine geringe Abweichung vom idealen kreisrunden Durchmesser eines Kanalausgangs auftritt.

Der Mittelpunkt eines Kanaleingangs kann im einfachsten Fall überhalb des Mittelpunkts eines Kanalausgangs liegen. Der Mikrokanal tritt dann sozusagen senkrecht durch das Glaselement. Es ist aber auch möglich, dass die Mittelpunkte von Kanaleingängen und Kanalausgängen gegeneinander versetzt sind, so dass der Mikrokanal einem Winkel durch das Glaselement tritt, der von der Orthogonalen durch die Glaselementfläche abweicht. Insbesondere geeignet sind Winkel von bis zu etwa 10° von dieser Orthogonalen, d.h Winkel in eine, Bereich von 0,1° bis 10°. Insbesondere ist es möglich, dass die Durchtrittswinkel der Mikrokanäle nicht überall im Glaselement die gleichen sind. Die lokale Einstellung der Durchtrittswinkel erlaubt es, die Kanalmatrix des Glaselements den Bedürfnisses anzupassen.

Für die Lager der Aufweitung der Kanalöffnung ist im Wesentlichen die Fokuslage verantwortlich. Wie beschrieben weitet der nachfolgende Ätzprozess die Schädigung im Glasmaterial auf. Je größer der Schädigungsbereich ist, desto größer ist auch die Aufweitung.

Um die Eigenschaften der Aufweitung des Kanaleingangs einzustellen wird die Form des langgestreckten Fokus ausgenutzt. Dieser ist nämlich so ausgestaltet, dass sich entlang der Strahlachse betrachtet an einen Bereich mit höchster Energie ein aufgeweiteter Bereich anschließt. Der langestreckte Fokusbereich wird so platziert, dass der aufgeweitete Anteil in einem Oberflächenbereich des Glaselement-Grundkörpers liegt.

Dieser wird beim späteren Ätzen zum aufgeweiteten Kanaleingang, insbesondere mit trichterförmiger Aufweitung. Der Ätzschritt wird vorteilhaft so ausgeführt wird, dass die Mikrokanäle zumindest im Bereich des Kanaleingang einen Durchmesser von 5 µm bis 200 µm aufweisen, bevorzugt von 7 µm bis 130 µm, besonders bevorzugt von 10 µm bis 100 µm.

Eine Variante des Verfahrens sie vor, dass filamentförmige Schädigungen zumindest eine Oberfläche des Glaselement-Grundkörpers nicht erreichen und die Öffnung zu dieser Oberfläche durch den nachfolgenden Ätzschritt erfolgt.

Dies liegt darin begründet, dass es von großem Interesse ist, auch eine möglichst genau Geometrie des Kanalausgangs bereit zu stellen, der insbesondere in einem zylinderförmigen Bereichs des Mikrokanals liegen kann. Um die Geometrie des Kanalausgangs einzustellen hat es sich gezeigt, dass sich die filamentförmige Schädigung vorteilhafter Weise nicht durch die Oberfläche des Glaselements hindurchtreten sollte, auf der sich der Kanalausgang befindet. Die filamentförmige Schädigung endet dann sozusagen unterhalb dieser Oberfläche. Auch dies wird durch die entsprechende Fokuslage eingestellt. Die Öffnung des Mikrokanals, d.h. der Kanalausgang, wird in diesem Fall durch den nachfolgenden Ätzschritt erzeugt.

Die Oberfläche des Glaselements-Grundkörpers kann mit Schleifen und Polieren behandelt werden, insbesondere vor und/oder nach dem Ätzen. Als besonders vorteilhaft hat sich das Schleifen und Polieren nach dem Ätzschritt erwiesen, da damit besonders ebene Oberflächen auch um den Kanaleingang und/oder Kanalausgang erzielen lassen.

In einem vorteilhaften Verfahren werden durch das Ätzen halbkugelförmige kalottenförmige Einpressungen Vertiefungen an der Kanalinnenwand eingebracht.

Dies erfolgt insbesondere dann, wenn ein langsamer Ätzprozess durchgeführt wird, insbesondere mit eiber Ätzrate von kleiner als 15 µm pro Stunde vorgesehen.

Es wird vermutet, dass die kalottenförmigen Vertiefungen durch Strukturen verursacht werden, die beim Einfügen der filamentförmigen Schädigungen auftreten. Als Ätzmedium ist eine flüssige Ätzlösung besonders vorteilhaft. Das Ätzen wird gemäß dieser Ausführungsform also nasschemisch durchgeführt. Dies kann ebenfalls günstig sein, um während des Ätzens Glasbestandteile von der Oberfläche zu entfernen.

Als Ätzlösungen können sowohl saure, als auch alkalische Lösungen verwendet werden. Als saure Ätzmedien sind insbesondere HF, HCl, H2SO4 , Amoniumbifluorid, HNO3-Lösungen oder Mischungen aus diesen Säuren geeignet. Für basische Ätzmedien kommen bevorzugt KOH- oder NaOH-Laugen in Betracht. Mit sauren Ätzlösungen lassen sich typischerweise größere Abtragsraten erzielen. Basische Lösungen werden aber bevorzugt, zumal ohnehin nur ein langsamer Abtrag angestrebt wird.

Das Ätzen wird vorteilhaft in einem Temperaturbereich von 40 °C bis 150 °C, insbesondere vorteilhaft von 50° bis 120 °C durchgeführt. Allgemein eignen sich für die erfindungsgemäße Strukturierung silikatische Gläser mit niedrigem Alkaligehalt besonders. Zu hohe Alkaligehalte erschweren das Ätzen. Gemäß einer Weiterbildung der Erfindung ist daher vorgesehen, dass das Glas des Glaselements ein Silikatglas mit einem Gehalt von Alkalioxiden kleiner als 17 Gewichtsprozent ist.

Das beschriebene Glaselement mit den Mikrokanälen kann vorteilhaft zur Untersuchung von biologischem Material, insbesondere menschlichen oder tierischen oder pflanzlichen Zellen und/oder Zellbestandteilen, insbesondere Proteinen und/oder DNA verwendet werden.

Alternativ zur ortsaufgelösten Diagnostik kann das Glaselement aufgrund seiner hohen Anzahl an Sacklöchern zur Einzelmoleküldiagnostik oder Single-Cell Diagnostik eingesetzt werden. Dabei wird eine insbesondere flüssige Probe in die Sacklöcher eingebracht, z.B. durch Verstreichen über einen Rakelprozesss oder über die oben genannten Prozesse. Dadurch gelangen - je nach Wahl der Konzentration - keine oder nur wenige bzw. genau ein einzelnes zu untersuchendes Molekül oder Zelle in ein Sackloch. Nun kann gezielt eine Vermehrung der zu untersuchenden Moleküle und/oder Zellen in den einzelnen Sacklöchern vorgenommen werden, um anschließend eine Analyse, insbesondere eine Fluoreszenzanalyse, durchzuführen.

Eine besonders vorteilhafte Ausführungsform sieht vor, dass in Kombination mit einer mikrofluidischen Zelle, die die einzelnen Sacklöcher oder Gruppen von

Sacklöchern gezielt ansteuern kann, spezifische Tests für jedes dieser Sacklöcher bzw. der Sacklochgruppe durchgeführt werden. Dies ist besonders für die medizinische Diagnostik von Vorteil, um die Heterogenität einer Probe zu bestimmen oder viele Verschiedene Tests auf kleinem Raum durchzuführen (High-Plex diagnostics).

Die Erfindung wird im Folgenden anhand der Figuren näher erläutert. Die Figuren und die zugehörigen Ausführungen repräsentieren Beispiele, die Erfindung ist nicht auf diese beschränkt. In den Figuren bezeichnen gleiche Bezugszeichen jeweils gleiche oder entsprechende Elemente. In einer Figur gezeigte Bezugszeichen können auch für andere Figuren gelten, auch wenn sie nicht dargestellt sind. Die Figuren zeigen schematische Ausführungsbeispiele, die Proportionen müssen nicht mit den Proportionen oder Abmessungen der erfindungsgemäßen Gegenstände übereinstimmen. Es zeigen:
Fig. 1a bis 1d: Verschiedene Ausführungsformen von Glaselementen mit aufgeweiteten Mikrokanälen im Schnitt.
Fig. 2: Den Schnitt durch einen Ausschnitt eines Glaselements mit aufgeweiteten Mikrokanälen.
Fig. 3: Die elektronenmikroskopische Aufnahme einer Wandung eines Mikrokanals.
Fig. 4a und Fig. 4b: Die Aufsicht auf die Ober- und Unterseite von Glaselementen.
Fig. 5: Die mikroskopische Aufnahme eines Schnitts durch ein Glaselement.
Fig. 6: Ein System zur ortsaugelösten Untersuchung von biologischem Material.
Fig. 7: Die Belegung von Farbstoffen auf der Oberseite eines Glaselements.
Fig. 8: Die Belegung von Farbstoffen in Sacklöchern eines Glaselements
Fig. 9: Eine Vorrichtung zur Laserbearbeitung der Glaselemente als Vorbereitung für ein nachfolgendes Ätzen.

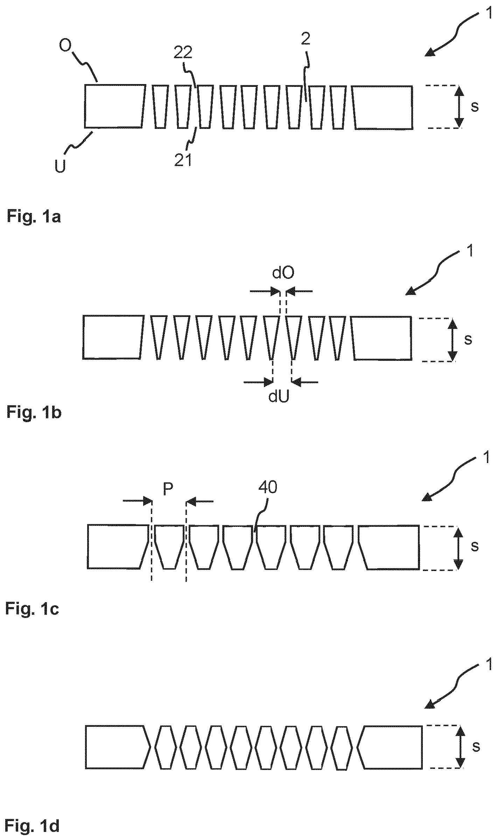

Fig. 1a zeigt eine Ausführungsform eines Glaselements (1) mit einer Vielzahl von Mikrokanälen (2). In diesem Ausführungsbeispiel verbinden die Mikrokanäle die Oberseite (O) des Glaselements mit dessen Unterseite (U) und treten durch diese hindurch. Wie zuvor beschrieben ist die Unterseite in der Regel diejenige Oberfläche bzw. Seite des Glaselements, welche der Probe abgewandt ist oder auf welcher die Analytik stattfindet. Die Unterseite ist in der Regel Oberfläche bzw. Seite des Glaselements, welche der Probe und damit dem zu untersuchenden biologischen Material zugewandt ist. Im Bereich der Unterseite (U) befindet sich in der Terminologie dieser Beschreibung der Kanaleingang (21) und im Bereich der Oberseite (O) der Kanalausgang (22).

In diesem Beispiel der Fig. 1a verjüngen sich die Mikrokanäle in Richtung von der Unterseite (U) zur Oberseite (O), in diesem Beispiel kontinuierlich. Allgemein wird die zumindest bereichsweise Aufweitung in dieser Beschreibung trichterförmig genannt. In dem gezeigten Beispiel erfolgt die Verjüngung kontinuierlich. Das Profil der Mikrokanäle (2) kann hier auch kegelstumpfförmig genannt werden. Der Bereich der Verjüngung erstreckt sich hier über die gesamte Dicke (s) des Glaselements.

Die Dicke oder Materialstärke (s) des Glaselements nach dessen Prozessierung kann wie beschrieben insbesondere von 0,1 mm bis 3 mm betragen. Das gezeigte Glaselement (1) weist im Wesentlichen planparallele Oberflächen (O) und (U) auf. Gleiches gilt üblicherweise für die Seitenflächen. Das Glaselement (1) ist vorteilhaft ein Slide.

In Fig. 1b wird ein ähnliches Ausführungsbeispiel dargestellt, in dem die Verjüngung der Mikrokanäle stärker ausgeprägt ist. Die Verjüngung in Richtung der Oberseite (O) und/oder die zumindest bereichsweise trichterförmige Ausbildung der Mikrokanäle (2) führt im Sinne der Erfindung dazu, dass der Durchmesser (dU) des Kanaleingangs (21) an der Unterseite (U) größer ist als der Durchmesser (dO) des Kanalausgangs (22) an der Oberseite (O) des Glaselements (1).

Fig. 1c zeigt eine weitere Ausführungsform, in welcher sich das Profil der Mikrokanäle aus einem sich verjüngenden, kegelstumpfförmigen Bereich im Bereich der Unterseite (U) und einem zylinderförmigen Bereich im Bereich der Oberseite (O) zusammensetzt. Die Gesamtform kann ebenfalls trichterförmig genannt werden. Der Mittelpunkt der Kanalausgänge (22) bestimmt wie zuvor beschrieben den Pitch (P). Ein möglichst geringer Pitch wird angestrebt, weil mit geringerem Pitch die Mikrokanaldichte zunimmt. Der Pitch kann wie beschrieben von 3 bis 70 µm betragen. Wie in diesem Beispiel gezeiht liegt der Mittelpunkt des Kanaleingangs (21) im Wesentlichen über dem Mittelpunkt des Kanalausgangs (22). D.h., beide Mittelpunkte liegen im Wesentlichen auf einer Achse, die senkrecht durch die Oberflächen (O) und (U) des Glaselements tritt.

Fig. 1d zeigt eine Ausführungsform, bei der sich Mikrokanäle (2) sowohl von der Unterseite (U) als auch von der Oberseite (O) in Richtung des Bulks des Glasmetarial verjüngen. In der hier beispielhaft gezeigten Form ergibt sich daraus eine Sanduhr- oder X-form. Diese ist hier im Prinzip aus zwei umgekehrt aufeinandergesetzten Kegelstümpfen zusammengesetzt, die sich an ihren Schmalseiten berühren.

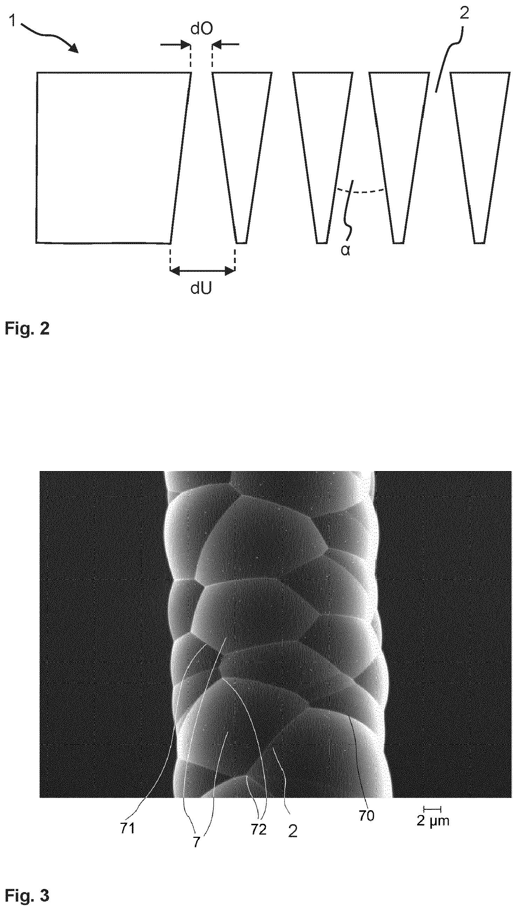

Fig. 2 zeigt einen Ausschnitt aus einem Glaselement (1) nach Fig. 1b. Eingezeichnet ist der Öffnungswinkel α, welcher als Winkel zwischen den Kanalwänden eines aufgeweiteten Bereichs eines Mikrokanals (2) aufzufassen ist. Vorteilhaft beträgt der Öffnungswinkel α von 0,1° bis 30°, besonders vorteilhaft von 2° bis 18°, wobei die Ober- und Untergrenzen in den Intervallen natürlich als vertauschbar aufzufassen sind.

Wie zuvor ausführlich erläutert ermöglichen es die sich verjüngenden Mikrokanäle dem Zielkonflikt zu lösen, dass einerseits ein möglichst geringer Pitch angestrebt wird, um die Auflösung der Untersuchung zu erhöhen sowie eine hohe Nachweisempfindlichkeit und somit ein großer Durchmesser des Kanaleingangs angestrebt wird, aber andererseits die mechanische Stabilität des Glaselements sichergestellt werden muss, um rationell anwendbar zu sein. In dem nicht verjüngten Bereich bleibt insbesondere so viel Material stehen, dass die mechanische Stabilität sichergestellt bleibt.

Fig. 3 zeigt die elektronenmikroskopische Aufnahme der Wandung eines Mikrokanals (2) eines erfindungsgemäßen von den Erfindern hergestellten Glaselements (1), und zwar im Ausschnitt eines Bereichs (40) eines Mikrokanals wie in Fig. 1c gezeigt. Die Querabmessung, beziehungsweise der Durchmesser des Kanals (2) liegt wie bereits gesagt hier kleiner als 200 Mikrometer, in diesem Beispiel etwa 14 Mikrometer. Ohne Beschränkung auf die hier beschriebenen Beispiele wird ein Durchmesser bevorzugt, der im Bereich von 5 Mikrometern bis 200 Mikrometern liegt.

Gut sichtbar sind die kalottenförmigen beziehungsweise abgerundeten, kappenförmigen Vertiefungen (7), die durch das Ätzen der filamentförmigen Schädigung entstehen, die durch den Ultrakurzpuls-Laserstrahl in das Glaselement eingebracht werden. Die Tiefe der kalottenförmigen Vertiefungen (7) ist typischerweise kleiner als 5 µm, bei Querabmessungen von typischerweise 5 bis 20 µm.

Durch den Ätzprozess der filamentförmigen Schädigungn grenzen die kalottenförmigen Vertiefungen (7) aneinander an, wobei die aneinander stoßenden konkaven Rundungen der Vertiefungen (7) Grate (70) ausbilden. Weiterhin ist zu erkennen, dass die Grate (70) in Aufsicht auf die Vertiefungen (7) gesehen polygonale Begrenzungslinien (71) der Vertiefungen (7) bilden. Dabei ist die mittlere Anzahl der Ecken (72) der Begrenzungslinien (71) der Vertiefungen (7) vorzugsweise auch kleiner als acht, vorzugsweise kleiner als sieben. Letzteres Merkmal ergibt sich dann, wenn die von den meisten kalottenförmigen Vertiefungen eingenommenen Gebiete im mathematischen Sinne konvex sind.

Die Grate (70) des in Fig. 3 gezeigten Kanals 2 sind sehr schmal, es gibt keine erkennbaren Bereiche, in denen die konkaven Wölbungen der Vertiefungen (7) über einen konvex gewölbten Bereich am Grat (70) ineinander übergehen. Die Struktur der Kanäle (2) kann daher gemäß einer Weiterbildung der Erfindung auch so beschrieben werden, dass der Flächenanteil konvex geformter Bereiche in einem Kanal (2) kleiner als 5%, vorzugsweise kleiner als 2% ist.

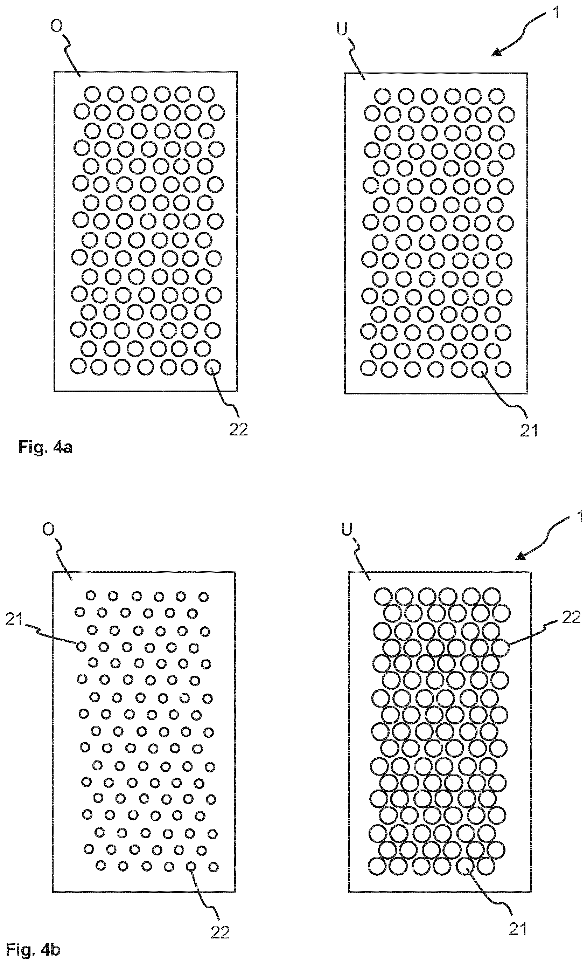

Fig. 4a stellt schematisch eine Aufsicht auf die Oberseite (O) und die Unterseite (U) eines Glaselements (1). Die Durchmesser von Kanaleingängen (21) und Kanalausgängen (22) sind im Wesentlichen gleich. Ein solches Muster ist beispielsweise bei einer Ausführungsform gemäß fig. 1d zu erwarten.

Fig. 4b zeigt eine analoge Aufsicht, die Durchmesser von Kanaleingängen (21) sind jedoch kleiner als die von Kanalausgängen (22). Ein solches Muster ist beispielsweise bei einer Ausführungsform gemäß den Fig. 1a bis 1c.

Wie in den Fig. 4a bis 4b gezeigt bilden die Kanaleingänge und -ausgänge eine Matrix. Weil die Position eines Kanaleingangs einer Position eines Kanalausgangs zugeordnet ist, kann bei Kenntnis der Lage des Kanalausgangs eine ortsaufgelöste Bestimmung eines Ereignisses, beispielsweise das Vorliegen von DNA oder bestimmten biologischen Materials, auf der Seite des Kanaleingangs und damit auf der Probenseite erfolgen.

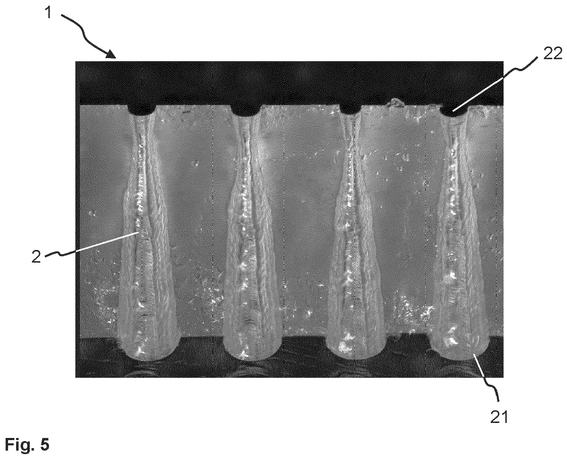

In Fig. 5 ist die durch ein optischen Mikroskop aufgenommene Fotografie des Schnitts durch ein von dem Erfindern hergestelltes Glaselement (1) abgebildet, welches im Wesentlichen der Ausführungsform nach Fig. 1c entspricht. An einen trichterförmig aufgeweiteten Bereich schließt sich ein zylinderförmiger Bereich der des Mikrokanals (2) an. Die Durchmesser des Kanaleingangs (21) and der Unterseite des Glaselement (1) ist größer als der Durchmesser des Kanalausgangs (22) an der Oberseite. In dem vorliegenden Ausführungsbeispiel wurde das SCHOTT Glas D263 verwendet. Die Erfindung ist nicht auf bestimmte Glassorten beschränkt. Andere Ausführungsbeispielen wurden mit Borosilikatgläsern und/oder alkalifreien Gläsern hergestellt. Insbesondere Borosilikatgläser werden aufgrund ihrer chemischen Beständigkeiten im Laborbereich häufig eingesetzt. Wie schon beschrieben sind insbesondere solche Gläser vorteilhaft, die eine geringe Eigenfluoreszenz im sichtbaren Bereich des Spektrums aufweisen.

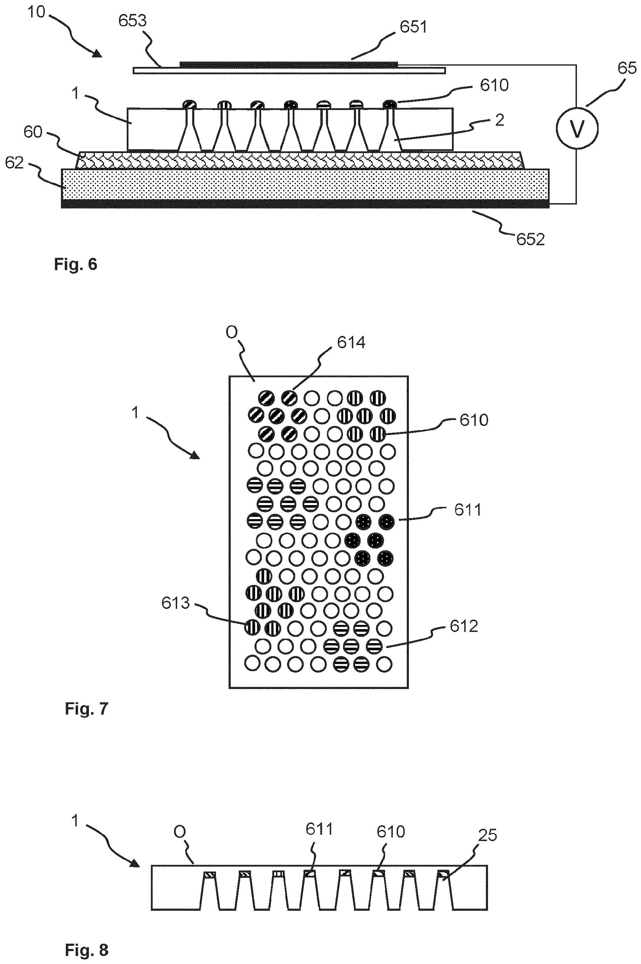

Fig. 6 zeigt ein exemplarisches System (10) zur ortsaufgelösten Bestimmung von biologischem Material (60). Das zu untersuchende biologische Material befindet sich hier auf einem Probenträger (62). Das Glaselement (1) mit den beschriebenen Mikrokanälen (2) befindet sich in Kontakt mit dem biologischen Material (60). Es ist natürlich ebenso möglich, dass das Glaselement (2) in Kontakt mit dem biologischen Material gebracht wird, ohne dass dieses auf einem Träger präpariert wird. Beispielsweise kann das Glaselement direkt in Kontakt mit zum Beispiel Gewebe oder einer Zellkultur gebracht werden.

In den Mikrokanälen (2) kann sich ein Gel befinden, insbesondere ein beschriebenes Gel. Dieses in der Fig. 6 nicht dargestellt. Auf der Oberseite des Glaselements (1) ist zumindest ein DNA- oder RNA-sensitiver Farbstoff (610) im Bereich des Kanalausgangs (22) angebracht. Wird biologisches Material (60) insbesondere mit Hilfe des Gels durch die Mikrokanäle (2) geleitet, gelangt es auf der Oberseite in Kontakt mit den Farbstoffen (610). Die typische Färbung des Farbstoffs kann dann auf das Vorhandensein oder je nach Farbstoff das Vorliegen eines bestimmten DNA-Strangs hinweisen. Wie beschrieben werden die Farbstoffe üblicherweise mit Anregungslicht bestrahlt und dann die typische Fluorszenz des Farbstoffs mit Hilfe einer Auswerteeinrichtung gemessen. Die Auswerteeinrichtung ist in dieser Figur aus Übersichtsgründen nicht dargestellt. Wie ebenfalls beschrieben kann dies insbesondere eine elektronische Kamera sein.

Vorteilhaft können an verschiedenen Kanalausgängen (22) verschiedene Farbstoffe (610) angebracht sein. Bei der Analyse von biologischem Material können dann verschiedene DNAs bzw. DNA-Stränge identifiziert werden.

In der Aufsicht ergibt sich in dem gezeigten System (10) ein Muster, aus dem ortsaufgelöst auf das Vorhandensein bestimmten biologischen Materials, insbesondere DNA, geschlossen werden kann. Alle für DNA getroffenen Aussagen gelten analog für RNA.

Der Transport von biologischem Material (60) in bzw. durch die Mikrokanäle (2) kann durch das Vorhandensein einer Transporteinrichtung (65) unterstützt werden. Im Beispiel der Fig. 6 ist dies eine Einrichtung, die allgemein ein elektrisches Potential zum Transport des biologischen Materials nutzt. Gezeigt ist eine Kondensatoreinrichtung, bei welcher eine Spannungsquelle (V) mit den Elektroden (651, 652) verbunden ist. Die Elektroden sind in diesem Beispiel auf der Unterseite des Probenträgers (62) und auf einer nichtleitenden Platte (653), insbesondere Glasplatte oder Kunststofff, angeordnet. Die Elektroden bilden somit einen Plattenkondensator, zwischen dessen Platten sich das biologische Material (62) und das Glaselement (1) befinden. Bei Anlegen von Spannung wird ein elektrisches Feld erzeugt, welches das biologische Material und/oder dessen umgebendes Medium in Richtung einer Elektrode (651) gezogen oder gedrückt wird.

Die elektrisch nicht leidende Platte (653) kann auch als Abschluss für die Mikrokanäle (2) dienen, wenn sie in Kontakt mit der Oberseite (O) des Glaselements gebracht wird. Dann entsteht sozusagen ein Sackloch und der Farbstoff wird durch das nachrückende biologische Material nicht von der Oberfläche des Glaselements entfernt.

Die Elektroden (651, 652) können rationell durch Beschichtung aufgebracht werden, insbesondere durch Sputtern.

Andere Konfigurationen sind ebenfalls möglich und von der Erfindung umfasst. Insbesondere ist es auch möglich, statt der elektromotorischen Kraft zum Transport des biologischen Materials Druck und/oder Unterdruck zu verwenden.

Fig. 7 zeigt die Aufsicht auf ein mit auf der Oberseite (O) mit sensitiven Farbstoffen (610, 611, 612, 613, 614) versehenes Glaselement (1), insbesondere in einem System nach Fig. 6. Das dargestellte Muster zeigt schematisch ein mögliches Untersuchungsergebnis.

Fig. 8 stellt zur Deutlichkeit noch einmal die Ausführungsform eines Glaselements mit Mikrokanälen (2) ausgebildet als Sackloch (25) dar. Am Boden der Sacklöcher sind die vorgenannten Farbstoffe (610, 611) angebracht. Die Elektrode (651) einer elektrischen Transporteinrichtung (65) kann einfach als auf der Oberfläche des Glaselements aufgebracht sein. Damit der Nachweis Farbstoff-Fluoreszenz durch den über dem Boden des Sacklochs befindlichen Glasmaterial möglich ist, handelt es sich in diesem Fall insbesondere um eine transparente Elektrode, die beispielsweise als ITO-Beschichtung dargestellt werden kann. Alternativ ist es auch möglich, die Elektrode als Raster und/oder Gitter auszubilden, insbesondere mit Leerstellen über dem Boden der entsprechenden Sacklöcher (25).

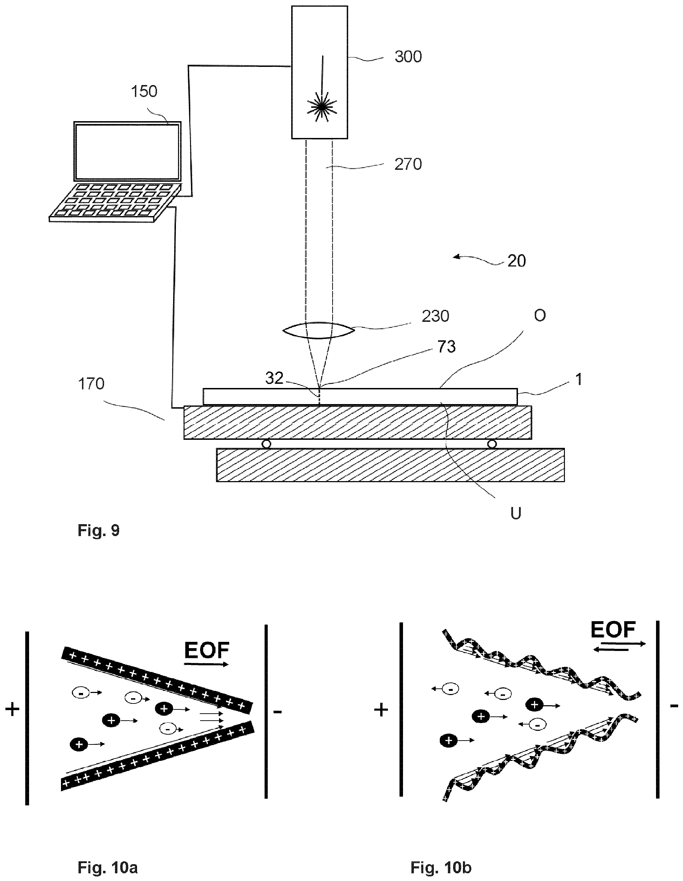

In Fig. 9 ist ein Ausführungsbeispiel für eine Laserbearbeitungsvorrichtung (20) gezeigt, mit welcher in ein Glaselement-Grundkörper (1) filamentförmige Schädigungen (32) eingefügt werden können, um nachfolgend in einem Ätzprozess Mikrokanäle an den Stellen der filamentförmigen Schädigungen (32) einzufügen. Die Vorrichtung (20) umfasst einen Ultrakurzpulslaser (300) mit vorgeschalteter Fokussierungsoptik (230) und eine Positioniereinrichtung (170). Mit der Positioniereinrichtung (170) kann der Auftreffpunkt (73) des Laserstrahls (270) des Ultrakurzpulslasers (300) auf der Oberfläche (O) eines zu bearbeitenden plattenförmigen Glaselements (1) lateral positioniert werden. Bei dem dargestellten Beispiel umfasst die Positioniereinrichtung (170) einen x-y-Tisch, auf dem das Glaselement (1) auf der Unterseite (U) aufliegt. Alternativ oder zusätzlich möglich ist aber auch, die Optik beweglich auszubilden, um den Laserstrahl (270) zu bewegen, so dass der Auftreffpunkt (32) des Laserstrahls (270) bei festgehaltenem Glaselement (1) bewegbar ist.

Fig. 10a und Fig 10b zeigen schematisch den Einfluss der Struktur der Mikrokana!wände. Während in Fig. 10a eine glatte Kanalwand dargestellt wird, ist in Fig. 10b eine strukturierte Kanalwand gezeigt, die eine Rauigkeit Ra aufweist. Die Kanalwände weisen einen Öffnungswinkel auf. Die Struktur kann die Form der kalottenförmigen Vertiefungen haben. EOF bezeichnet den elektroosmotischen Flusses. Der elektroosmotische Fluss ist wie geschrieben eine durch Ladungen induzierte Bewegung von Flüssigkeiten entlang einer polaren Oberfläche in einem elektrischen Feld. Eine größere Rauigkeit der polaren Oberfläche entsprechend Fig. 10b führt zu einer Verringerung der Geschwindigkeit des EOF und eventuellen Umkehr der Bewegungsrichtung des polaren biologischen Materials im Glaselement im Vergleich zu einer Oberfläche mit geringerer Rauigkeit entsprechend Fig. 10a.

Die Fokussierungsoptik (230) fokussiert den Laserstrahl (270) nun zu einem in Strahlrichtung, also dementsprechend quer, insbesondere senkrecht zur bestrahlten Oberfläche (O) langgezogenen Fokus. Ein solcher Fokus kann beispielsweise mit einer kegelförmigen Linse (ein sogenanntes Axikon) oder einer Linse mit großer sphärischer Aberration und/oder großer chromatischer Aberration erzeugt werden. Die Steuerung der Positioniereinrichtung (170) und des Ultrakurzpulslasers (300) wird vorzugsweise mittels einer programmtechnisch eingerichteten Recheneinrichtung (150) durchgeführt. Auf diese Weise lassen sich vorbestimmte Muster von lateral entlang der Oberfläche (O) verteilten filamentförmigen Schädigungen (32) erzeugen, dies insbesondere durch Einlesen von Positionsdaten, vorzugsweise aus einer Datei oder über ein Netzwerk. Durch die Lage des Fokus kann die Form der filamentförmigen Schädigungen (32) so eingestellt werden, dass eine lokale Aufweitung der durch das spätere Ätzen erzeugten Mikrokanäle (2) resultiert.

Gemäß einem Ausführungsbeispiel können folgende Parameter für den Laserstrahl verwendet werden: Die Wellenlänge des Laserstrahls beträgt 1064nm, typisch für einen YAG-Laser. Es wird ein Laserstrahl mit einem Rohstrahldurchmesser von 12 mm erzeugt, der dann mit einer Optik in Form einer Bikonvex-Linse mit einer Brennweite von 16 mm fokussiert wird. Die Pulsdauer des Ultrakurzpulslasers beträgt weniger als 20 ps, bevorzugt etwa 10 ps. Die Pulse werden in Bursts mit 2 oder mehr, bevorzugt 4 oder mehr Pulsen abgegeben. Die Burstfrequenz beträgt 12 - 48ns, vorteilhaft etwa 20ns, die Pulsenergie mindestens 200 Mikrojoule, die Burstenergie entsprechend mindestens 400 Mikrojoule.

Anschließend, nach dem Einfügen einer oder insbesondere einer Vielzahl von filamentförmigen Schädigungen (32) wird das Glaselement (1)1 entnommen und in einem Ätzbad gelagert, wo in einem Ätzprozess Glas entlang der filamentförmigen Schädigungen (32) entfernt wird, so dass an der Stelle einer solchen Schädigung (32) jeweils ein Mikrokanal (2) in das Glaselement 1 eingefügt wird. Bevorzugt wird ein basisches Ätzbad mit einem ph-Wert >12, beispielsweise eine KOH-Lösung mit > 4mol/l bevorzugt > 5mol/l besonders bevorzugt > 6mol/l, aber <30mol/l. Das Ätzen wird gemäß einer Ausführungsform der Erfindung unabhängig vom verwendeten Ätzmedium bei einer Temperatur des Ätzbads von >70°C, bevorzugt >80°C, besonders bevorzugt >90°C durchgeführt.

Das Glaselement sowie das System gemäß der Erfindung weisen den Vorteil auf, dass sie eine ortsaufgelöste Untersuchung von biologischem Material ermöglichen. Das Glaselement ist rationell herstellbar und besteht aus Materialien, die im Laborumfeld gebräuchlich sind. Nach Verwendung ist das Glas wieder einschmelzbar, womit die Rohstoffe wiederverwertbar sind.

## Patentansprüche

1. Glaselement (1) zur Aufnahme und/oder Weiterleitung von biologischem Material (60), insbesondere menschlichen oder tierischen oder pflanzlichen Zellen und/oder Zellbestandteilen, insbesondere Proteinen und/oder DNA und/oder RNA,
mit einer Vielzahl von Mikrokanälen (2), welche bevorzugt eine Oberfläche (U, O) des Glaselements (1) mit der gegenüberliegenden Oberfläche (O, U) des Glaselements (1) verbinden oder in einem Sackloch (25) enden,
wobei sich Mikrokanälen (2) von der Unterseite (U) in Richtung zur Oberseite (O) verjüngen;
bevorzugt sind Mikrokanäle (2) zumindest bereichsweise trichterförmig ausgebildet.

2. Glaselement (1) nach Anspruch 1, wobei der Durchmesser (dU) des Kanaleingangs (21) eines Mikrokanals an der Unterseite (U) größer als der Durchmesser (dO) des Kanalausgangs (22) oder Kanalendes (25) im Bereich der Oberseite (O) ist.

3. Glaselement (1) nach mindestens einem der voranstehenden Ansprüche, wobei die Mikrokanäle (2) zumindest im Bereich eines Kanaleingangs (21) oder Kanalausgangs (22) oder Kanalendes (25) des Glaselements (1) einen Durchmesser (dU, dO) von 5 µm bis 200 µm aufweisen, bevorzugt von 7 µm bis 130 µm, besonders bevorzugt von 10 µm bis 100 µm; bevorzugt beträgt der Öffnungswinkel (α) des trichterförmigen Bereichs von 0,1° bis 30°, besonders bevorzugt von 2° bis 18°.

4. Glaselement (1) nach mindestens einem der voranstehenden Ansprüche, wobei sich an den trichterförmigen Bereich im Bereich des Kanaleingangs (21) ein zylinderförmiger oder trichterförmiger Bereich insbesondere im Bereich des Kanalausgangs (22) oder Ende des Sacklochs (25) anschließt.

5. Glaselement (1) nach mindestens einem der voranstehenden Ansprüche, wobei die Mikrokanaldichte mindestens 20 / mm² beträgt, insbesondere mindestens 400 / mm², insbesondere mindestens 10000 / mm², insbesondere jeweils bis höchstens 16000 / mm².

6. Glaselement (1) nach mindestens einem der voranstehenden Ansprüche, wobei die Kanalwand der Mikrokanäle (2) eine Struktur mit einer Vielzahl von abgerundeten, kalottenförmigen Vertiefungen (7) aufweist, die insbesondere eine Tiefe von weniger als 5 µm aufweisen.

7. Glaselement (1) nach mindestens einem der voranstehenden Ansprüche, wobei sich im Bereich der Kanalausgänge (22) oder am Boden der Sacklöcher (25) zumindest ein DNA- oder RNA-sensitiver Farbstoff (610, 611, 612, 613, 614) befindet.

8. Glaselement (1) nach mindestens einem der voranstehenden Ansprüche, wobei sich im Bereich der Kanalausgänge (22) oder am Boden der Sacklöcher (25) Moleküle zur Polymerasekettenreaktion befinden , insbesondere DNA, Primer, Nukleotide und/oder das Enzym DNA-Polymerase.

9. System (10) zur Untersuchung von biologischem Material (60), insbesondere menschlichen oder tierischen oder pflanzlichen Zellen und/oder Zellbestandteilen, insbesondere Proteinen und/oder DNA und/oder RNA, umfassend ein Glaselement (1) mit einer Vielzahl von Mikrokanälen (2), insbesondere ein Glaselement nach mindestens einem der Ansprüche 1 bis 8,
wobei das Glaselement (1) im Betriebszustand zur Aufnahme und/oder Weiterleitung des biologischen Materials (60) in den oder durch die Mikrokanäle (2) von der Unterseite (U) des Glaselements in den Bereich der Oberseite (O) des Glaselements dient,
mit einer Auswerteeinrichtung, mit welcher im Betriebszustand das biologische Material untersuchbar ist.

10. System (10) nach Anspruch 9 wobei sich im Bereich der Kanalausgänge (22) oder am Boden der Sacklöcher (25) zumindest ein DNA- oder RNA-sensitiver Farbstoff (610, 611, 612, 613, 614) befindet, dessen Farbinformation durch die Auswerteeinrichtung erfassbar ist, insbesondere ortsaufgelöst erfassbar.

11. System (10) nach mindestens einem der Ansprüche 9 bis 10, wobei dem Glaselement (2) eine Transporteinrichtung (65) zugeordnet ist, mit welcher das zu untersuchende biologische Material (60) im Betriebszustand in die Mikrokanäle (2) des Glaselements (1) eingebracht wird und/oder durch diese transportiert wird.

12. System nach Anspruch 11, wobei die Transporteinrichtung (65) eine Unterdruckeinheit umfasst, mit welcher im Betriebszustand Unterdruck an die Ausgangsseite des Glaselements angelegt wird und/oder eine Einrichtung, um ein Potentialgefälle insbesondere über die Dicke des Glaselement zu erzeugen, so dass zu untersuchendes biologisches Material (60) durch einen Fluss oder durch ein elektrisches Feld in die Mikrokanäle (2) eingebracht und/oder durch diese transportiert wird, und/oder eine Rakeleinheit, mit welcher zu untersuchendes biologisches Material durch Verstreichen in die Sacklöcher eingebracht wird.

13. System (10) nach mindestens einem der Ansprüche 9 bis 12, wobei im Betriebszustand biologisches Material in Mikrokanälen (2) und/oder Sacklöchern (25) vervielfältigt wird, insbesondere sind im Bereich der Kanalausgänge (22) oder am Boden der Sacklöcher (25) Moleküle zur Polymerasekettenreaktion vorhanden.

14. System (10) nach mindestens einem der Ansprüche 9 bis 13, wobei die Auswerteeinrichtung eine geschlossene oder nanoporöse Membran insbesondere aus einem Nichtleiter oder einem Halbleiter oder der Kombination aus nichtleitenden, leitenden und/oder halbleitenden Bestandteilen insbesondere Glas, Silizium, Graphen und/oder Biomolekülen insbesondere Lipidmolekülen oder Transmembranproteine auf der Ausgangsseite der Mikrokanäle (2) umfasst.

15. Verfahren zur Herstellung eines Glaselements (1) mit einer Vielzahl von Mikrokanälen (2), welche eine Oberfläche (U, O) des Glaselements mit der gegenüberliegenden Oberfläche (O, U) des Glaselements verbinden, insbesondere eines Glaselements (1) nach mindestens einem der Ansprüche 1 bis 7, umfassend die Verfahrensschritte
- Bereitstellen eines Ultrakurzpuls-Lasers (300),
- Bereitstellen eines Glaselement-Grundkörpers (1),
- ein gepulster Laserstrahl (270) des Ultrakurzpulslasers (300) wird auf den Glaselement-Grundkörper (1) gerichtet, wobei
- die Wellenlänge des Laserstrahls (270) und das Material des Glaselement-Grundkörpers (1) so aufeinander abgestimmt sind, dass das Werkstück für den Laserstrahl (270) im Wesentlichen transparent ist, und wobei
- der Laserstrahl (270) zu einem in Strahlrichtung langgestreckten Fokusbereich (73) fokussiert wird, welcher mindestens teilweise in nerhalb des Glaselement-Grundkörpers (1) liegt, wobei
- die Intensität des Laserstrahls (270) und die Ausdehnung des Fo kus so groß sind, dass der Laserstrahl (270) im Werkstück eine fila mentförmige Schädigung (32) hinterlässt, und wobei eine Vielzahl solcher filamentförmiger Schädigungen (32) eingefügt werden,
- Aufätzen der filamentförmigen Schädigungen (32) zu Mikrokanälen (2).

16. Verfahren nach Anspruch 15, wobei filamentförmige Schädigungen (32) zumindest eine Oberfläche (O) des Glaselement-Grundkörpers (1) nicht erreichen und die Öffnung zu dieser Oberfläche (O) durch den nachfolgenden Ätzschritt erfolgt.

17. Verfahren nach mindestens einem der Ansprüche 15 bis 16, wobei durch das Ätzen kalottenförmige Vertiefungen (7) an der Kanalinnenwand der Mikrokanäle (2) eingebracht werden.
